# EUROPEAN PATENT SPECIFICATION

(11) **EP 3 171 942 B1**
(45) Date of publication and mention of the grant of the patent: **17.04.2019**
(21) Application number: 15741277.6
(22) Date of filing: 16.07.2015
(51) Int. Cl.: A61Q 5/12, A61K 8/86

(54) **HAIR CONDITIONING COMPOSITIONS**
HAARPFLEGEZUSAMMENSETZUNGEN
COMPOSITIONS APRES-SHAMPOING

(30) Priority: 22.07.2014 GB 201412976
(43) Date of publication of application: 31.05.2017
(73) Proprietor: Perachem Limited, Yeadon, Leeds LS19 7DP (GB)
(72) Inventor: LEWIS, David Malcolm, Otley Yorkshire LS21 2AL (GB); HAWKES, Jamie Anthony, Leeds Yorkshire LS20 9PS (GB); MAMA, John, Leeds Yorkshire LS8 2JB (GB)
(74) Representative: Appleyard Lees IP LLP
(86) International application number: PCT/GB2015/052067
(87) International publication number: WO 2016/012757

(56) References cited:
- WO-A1-2004/093835
- WO-A2-2010/141683
- CN-A- 103 301 034
- DE-U1-202008 016 055
- FR-A1- 2 920 972
- US-A- 5 474 712
- US-A1- 2004 116 311
- US-A1- 2011 256 249
- DATABASE GNPD [Online] MINTEL; January 2014 (2014-01), Anonymous: "Detangling Conditioner", XP55213467, retrieved from Mintel accession no. 2289894 Database accession no. 2289894
- DATABASE GNPD [Online] MINTEL; June 2014 (2014-06), anonymous: "Resistance Hair Conditioner", XP55213471, retrieved from Mintel accession no. 2459329 Database accession no. 2459329
- DATABASE GNPD [Online] MINTEL; March 2014 (2014-03), Anonymous: "Conditioner", XP55213474, retrieved from Mintel accession no. 2322677 Database accession no. 2322677

## Description

The present invention relates to the treatment of hair to improve its condition, for example the softness and handling of the hair.

The application of agents to human hair to improve its condition has been known for many years, and consumers commonly apply a conditioning composition to hair after shampooing in order to improve the handle and feel of the hair. The active ingredients in most commercially available home-use conditioning compositions include quaternary ammonium salts and silicones. Water soluble esters, for example polysorbate 80 have on occasion been used ingredient in hair treatment compositions, for example shampoo and conditioning compositions. However these compounds are typically included in low concentrations as surfactants or emulsifiers.

The present inventors have found that certain water soluble esters can provide excellent hair conditioning, with long lasting efficacy.

According to a first aspect of the present invention there is provided the use of a composition comprising from 20 to 45 wt% of a water soluble ester of formula RCOXR' as a hair conditioning agent, wherein the hair conditioning agent is an ester of ethoxylated sorbitan and a fatty acid having from 10 to 30 carbon atoms which includes from 10 to 50 ethoxy groups.

The present invention provides the use of an ester of an alkoxylated alcohol as a conditioning agent.

Suitably the presence of alkoxy residues provides water solubility.

The hair conditioning agent may be an ester of a monocarboxylic acid, a dicarboxylic acid or a polycarboxylic acid, i.e. the group R may include one or more carboxylic acid groups or the residue of one or more carboxylic acid groups.

In embodiments in which the hair conditioning agent is an ester of a dicarboxylic acid, one or both of the acid groups may be esterified.

In embodiments in which the hair conditioning agent is an ester of a polycarboxylic acid, one, some or all of the acid groups may be esterified.

In preferred embodiments the hair conditioning agent is an ester of a monocarboxylic acid.

In especially preferred embodiments R is an optionally substituted alkyl or alkenyl group.

Suitably R is an optionally substituted alkyl or alkenyl group having from 9 to 30 carbon atoms, preferably from 11 to 25 carbon atoms.

In some embodiments R is an optional substituted alkyl or alkenyl group having from 9 to 13 carbon atoms.

Preferably R is an optionally substituted alkyl or alkenyl group having from 13 to 21 carbon atoms, and most preferably from 15 to 19 carbon atoms.

Suitably the hair conditioning agent may be selected from an ester of stearic acid, lauric acid or oleic acid. In some embodiments the conditioning agent may be an ester of a mixture of acids, for example it may be an ester of coconut acid.

Preferably R is an unsubstituted alkyl or alkenyl group. More preferably R is an unsubstituted alkenyl group. Most preferably R is an unsubstituted alkenyl group including a single double bond.

In some especially preferred embodiments the hair conditioning agent is an alkoxylated alcohol ester of a monounsaturated carboxylic acid having from 12 to 24 carbon atoms, preferably from 16 to 20 carbon atoms.

Suitably the hair conditioning agent is an ester of an acid selected from myristoleic acid, palmitoleic acid, sapienic acid, oleic acid, elaidic acid, vaccenic acid, gadoleic acid, erucic acid and nervonic acid.

In especially preferred embodiments the hair conditioning agent is an ester of oleic acid.

The hair conditioning agent may comprise a mixture of compounds in which one or more different hydroxy groups are esterified.

In preferred embodiments only one of the hydroxy groups of the alcohol is esterified.

Preferably the hair conditioning agent of the present invention is an ester of a polyalkoxylated alcohol.

The hair conditioning agent is an ester of a polyhydric alcohol.

The hair conditioning agent is an ester of a polyalkoxylated polyhydric alcohol.

The hair conditioning agent is an ester of an alcohol having 4 hydroxy groups.

Preferably the hair conditioning agent is an ester of an alcohol having from 10 to 50 alkoxy groups, preferably from 12 to 30 alkoxy groups, preferably from 14 to 26 alkoxy groups, more preferably from 16 to 24 alkoxy groups, most preferably from 18 to 22 alkoxy groups. In especially preferred embodiments preferably the conditioning agent is an ester of an alkoxylated alcohol having 20 alkoxy groups.

The skilled person will appreciate that for polyalkoxylated compounds the above amounts will refer to the average number of alkoxy groups per molecule of the compound. However commercial sources of these compounds will typically contain mixtures of compounds comprising different numbers of alkoxy groups.

In preferred embodiments the hair conditioning agent is an ester of an alcohol having from 4 hydroxy groups and from 10 to 30 alkoxy groups, preferably ethoxy groups.

The conditioning agent of the present invention is preferably an ester of an alkoxylated alcohol and a fatty acid, i.e. an acid of formula RCOOH wherein R is an optionally substituted alkyl or alkenyl group having at least 10 carbon atoms.

In especially preferred embodiments the hair conditioning agent is an ester of a monounsaturated carboxylic acid having 16 to 20 carbon atoms and a polyethoxylated polyhydric alcohol having 4 hydroxy groups and 15 to 25 ethoxy groups.

The hair conditioning agent is an ester of alkoxylated sorbitan.

Suitably the hair conditioning agent is an ester of alkoxylated sorbitan and an acid selected from stearic acid, lauric acid and oleic acid.

The hair conditioning agent is an ester of ethoxylated sorbitan and a fatty acid having from 10 to 30, preferably from 12 to 24 carbon atoms which includes from 10 to 50, preferably from 15 to 25, most preferably 20 ethoxy groups.

Most preferably the hair conditioning agent is the compound of formula (I): 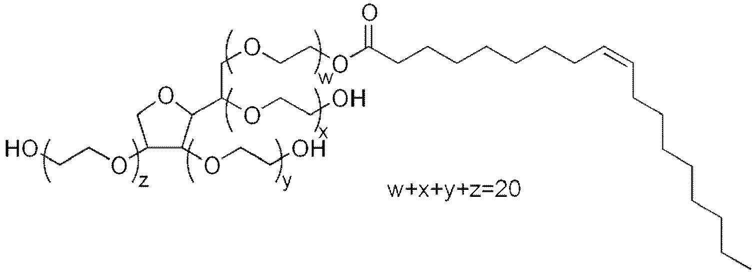 wherein w+x+y+z = 20

This compound is commonly known as polysorbate 80 and has been commercially available for many years. However it has not previously been used as a conditioning agent for hair.

The skilled person will appreciate that the commercial sources of the above compound will contain a mixture of compounds. For example it is clear from the formula that w, x, y and z may vary and thus different isomers may be present. The composition may also comprise mixtures in which different hydroxy groups are esterified. Commercial sources may also include small amounts of diester and triester and residual free alcohol or acid.

However in preferred embodiments the conditioning agent of the present invention consists essentially of the isomers of the compound of formula (I).

Other compounds useful as hair conditioning agents according to the present invention include those known as polysorbate 20, polysorbate 60 and polysorbate 65.

Polysorbate 80 is a very safe compound commonly used in the cosmetic, pharmaceutical and food industries. In cosmetic applications it has previously been included as a surfactant or emulsifier. However formulators have been reluctant to include this component at levels greater than 2 wt% in cosmetic preparations as it is known to interfere with surfactant micelle formation and can lead to foaming problems.

The present inventors have surprisingly found that application of a composition comprising an ester of formula RCOXR' improves the condition of hair.

The improvement in condition may be an increase in the softness of the hair.

The improvement in condition of the hair may be an improvement in ease of handling of the hair.

The improvement in condition may be a perceived decrease in the level of static electricity on the hair.

The improvement in condition may be a perceived increase in volume of the hair.

The improvement in condition of the hair may be a reduction in tangling of the hair.

The improvement in condition of the hair may be regarded as an improvement in shine of the hair.

The improvement in condition of the hair may be an increase in the strength of the hair.

The improvement in condition of the hair may be perceived as an increase in the ease of styling and "cuttability" of the hair.

Thus the use of the conditioning agent of the present invention may provide a conditioning effect selected from one or more of an increase in softness, ease of handling, a reduction in static, a reduction in tangling, an increase in the ease of styling and "cuttability", increased shine, increased volume and increased strength.

Preferably the present invention provides a durable hair conditioning agent. By durable we mean that the conditioning effect achieved by the conditioning agent has long lasting efficacy, and is not readily removed by washing/rinsing the hair.

Preferably the conditioning effect achieved by the present invention is durable to washing. Suitably the conditioning effect remains noticeable after normal shampooing of the hair. Preferably the conditioning effect remains following multiple shampooings of the hair for example after 2, 3, 4 or even 5 washes.

Without wishing to be bound by theory it is believed that the conditioning agent penetrates the hair fibre via the cuticle edge and remains within the fibre after shampooing. This theory is supported by the lack of surface residue from Scanning Electron Microscopy (SEM) and Energy Dispersive X-ray Spectroscopy (EDX) analysis (see example 4).

Without wishing to be bound by theory it is believed that the improvement in condition of the hair is achieved by the bonding of the fatty acid portion of the fatty acid portion of the ester to the hair keratin via a thioester linkage or an amide linkage.

Suitably the conditioning effect achieved remains noticeable to the user for at least 24 hours after contacting the hair with the conditioning agent, suitably for at least 48 hours, preferably for at least 72 hours. Users report that the conditioning effect achieved by the present invention is still noticeable a week after the hair has been contacted with the conditioning agent.

Disclosed herein is a method of treating hair, the method comprising contacting the hair with a conditioning composition comprising an ester of formula RCOXR' as defined in relation to the first aspect.

Preferred features of the method are as defined in relation to the first aspect. Thus the ester is preferably as defined in the first aspect and the method preferably provides a conditioning effect as defined in relation to the first aspect.

Disclosed herein is a hair conditioning composition comprising at least 5 wt% of an ester of formula RCOXR' as defined in relation to the first aspect.

Disclosed herein is the use of a conditioning composition as defined herein to improve the condition of hair contacted with the composition. The improvement in condition may be as defined in relation to the first aspect.

The conditioning composition comprises 20 to 45 wt% of the conditioning agent.

The composition may comprise a mixture of ester conditioning agents. In such embodiments the above amounts refer to the total of all such components present in the composition. For example the ester may be formed by reaction of an alcohol or thiol with a mixture of fatty acids. The ester may be formed by reaction of an acid with a mixture of alcohols or thiols. The ester may be formed by the reaction of a mixture of acids with a mixture of alcohols or thiols. The ester may be formed by the reaction of an acid with a polyhydric alcohol. In such embodiments mixtures may result from reaction of the acid with different hydroxy groups. Mixtures in which more than one hydroxy group is esterified may also be present. Thus the conditioning agent may comprise a mixture of monoesters, diesters, triesters etc. If the ester is formed from a dicarboxylic acid or polycarboxylic acid, one or more of the acid groups may be esterified and mixtures in which different acid groups are esterified may be present. The conditioning agent may also comprise a mixture of preformed esters.

The conditioning agent preferably comprises a solvent. Suitable solvents include water and other water miscible, cosmetically approved solvents, for example ethanol, glycerol, isopropanol and propylene carbonate. Preferably water is the major solvent and is present in a greater amount than any other solvent. Suitably water accounts for at least 50 wt% of all solvents present in the composition, preferably at least 60 wt%, more preferably at least 70 wt%, preferably at least 80 wt%, more preferably at least 90 wt%, preferably at least 95 wt% or at least 98 wt%. Suitably water is the only solvent present in the conditioning composition.

Preferably the conditioning composition comprises at least 10 wt% water, preferably at least 20 wt%, suitably at least 30 wt%, preferably at least 40 wt%, suitably at least 50 wt%, for example at least 55 wt%.

The conditioning composition may comprise up to 95 wt% water, preferably up to 90 wt%, more preferably up to 80 wt%, for example up to 75 wt%.

The conditioning composition of the present invention preferably comprises an ester of formula RCOXR' and water. It may optionally comprise one or more further components. Suitable further components include fragrances, swelling agents, preservatives, antioxidants, dyes, surfactants, chelating agents, traditional conditioning agents, shine agents, thiols, sulfites, thioglycolates, antistatic agents, pH modifiers and pH buffers.

Suitable swelling agents include urea.

Suitable pH modifiers include 2-amino-2-methylpropan-1-ol, sodium hydroxide, potassium hydroxide and ethanolamine.

The conditioning composition of the present invention preferably comprises an ester of formula RCOXR', water and one or more optional components. The one or more optional components are suitably present in a total amount of less than 20 wt%, preferably less than 10 wt%, more preferably less than 5 wt%.

Suitably the ester conditioning agent and water together comprise at least 70 wt% of the conditioning composition, preferably at least 80 wt%, more preferably at least 85 wt%, preferably at least 90 wt%, suitably at least 93 wt% or at least 95 wt%.

The conditioning composition suitably comprises an ester of formula RCOXR', water and a fragrance.

Suitable fragrances are cosmetically approved fragrances. These will be known to the person skilled in the art.

The conditioning composition suitably comprises an ester of formula RCOXR', water and a preservative.

Suitable preservatives include phenoxyethanol, ethylhexylglycerin, methylchloroisothiazolinone, methylisothiazolinone, 2-bromo-2-nitropropane-1,3-diol, benzyl alcohol, potassium sorbate, sodium benzoate and dehydroacetic acid. Other cosmetically approved preservatives may also be used. Such compounds are known to the person skilled in the art.

The conditioning composition of the present invention may comprise an ester of formula RCOXR', water, a fragrance and a preservative.

Because the conditioning agent of the present invention provides a significant improvement in condition of the hair, it is not necessary to include high levels of traditional conditioning agents such as quaternary ammonium compounds and silicones.

The conditioning composition preferably comprises less than 5 wt% silicone compounds, preferably less than 3 wt%, suitably less than 1 wt%, preferably less than 0.5 wt%, for example less than 0.1 wt% - or less than 0.01 wt%.

The conditioning composition preferably comprises less than 5 wt% quaternary ammonium compounds, preferably less than 3 wt%, suitably less than 1 wt%, preferably less than 0.5 wt%, for example less than 0.1 wt% or less than 0.01 wt%.

The conditioning composition of the present invention preferably comprises less than 10 wt% anionic surfactants, preferably less than 5 wt%, preferably less than 1 wt%, preferably less than 0.1 wt%, more preferably less than 0.01 wt%.

The conditioning composition of the present invention preferably comprises less than 10 wt% cationic surfactants, preferably less than 5 wt%, more preferably less than 1 wt%, preferably less than 0.1 wt%, most preferably less than 0.01 wt%.

The conditioning composition of the present invention preferably comprises less than 10% wt% amphoteric surfactants, preferably less than 5 wt%, more preferably less than 1 wt%, preferably less than 0.1 wt%, most preferably less than 0.01 wt%.

In the method of the present invention the conditioning composition is suitably applied to the hair, rubbed into the hair, optionally combed into the hair and left on the hair for a period of from 0.1 to 30 minutes. Suitably the hair conditioning composition is then rinsed from the hair. The conditioning agent is suitably applied to the hair in the manner similar to a shampoo or standard home use conditioner. Thus the amount needed will depend on the length and thickness of a user's hair but suitably sufficient composition is used to coat all of the hair.

Unlike traditional conditioning compositions the composition of the present invention is preferably applied to the hair prior to shampooing the hair. However embodiments in which the hair is shampooed first are also within the scope of the invention.

Thus in preferred embodiments the method of the present invention comprises contacting the hair with a conditioning composition comprising an ester of formula RCOXR', rinsing the hair and shampooing the hair.

As mentioned above the conditioning effect achieved by the method of the present invention is durable to washing.

The conditioning agent of the present invention has been shown to provide a significant improvement in the condition of the hair after just one use. However after continued use the condition of hair improves even further. In order to maintain excellent condition it is preferred that the conditioning agent is applied to the hair at least once every 4 to 10 washes, for example at least once every 7 to 10 washes.

The method of the present invention preferably provides a more durable improvement in condition of the hair than compositions of the prior art comprising silicone compounds.

The method of the present invention preferably provides a more durable improvement in the condition of the hair than a composition comprising a quaternary ammonium compound.

The invention will now be further defined with reference to the accompanying examples.

### Example 1

A conditioning composition was prepared comprising the following ingredients.

| | |
|---|---|
| • Water | 59% |
| • Polysorbate 80 | 40% |
| • Fragrance | 1% |

A tress of level 6 light brown hair was treated with the aforementioned conditioning composition as follows.
1. Apply a generous amount of the composition to the hair.
2. Massage into the hair and leave for 5 minutes.
3. Rinse off.
4. Shampoo.
5. Dry the hair using a hair drier.

After treating the hair in this manner, the hair had increased shine when compared to a comparative sample (which was shampooed only). The hair felt residue free. The treated hair was also easier to comb and "sat down" better than the comparative sample - indicating better manageability.

### Example 2

The composition of Example 1 was applied to male & female human heads using the method described in Example 1
1. Apply a generous amount of the composition to the hair.
2. Massage into the hair and leave for 5 minutes.
3. Rinse off.
4. Shampoo.
5. Dry the hair using a hair drier.

After treating the hair in this manner, both models agreed that the hair had improved shine and that it was softer and more manageable than before the treatment.

The male model noted that his hair sat much flatter on his head rather than being wavy.

Both models noticed that the manageability & softness were long lasting and noticed that the effects were still observed 1 week after application including several subsequent shampoos. Both models also agreed that there was no oily residue on the hair after treatment.

### Example 3

The composition of Example 1 was applied to male & female human heads using the method described in Example 1
1. Apply a generous amount of the composition to the hair.
2. Massage into the hair and leave for 5 minutes.
3. Rinse off.
4. Shampoo.
5. Rinse off.
6. Apply a conditioner
7. Dry the hair using a hair drier.

After treating the hair in this manner, both models agreed that the hair had improved shine and that it was softer and more manageable than before the treatment.
The female model noted that her hair was far softer and shinier than ever before.

Both models noticed that the manageability & softness were long lasting and noticed that the effects were still observed 10 days after application including numerous subsequent shampoos. Both models also agreed that there was no oily residue on the hair after treatment.

### Example 4

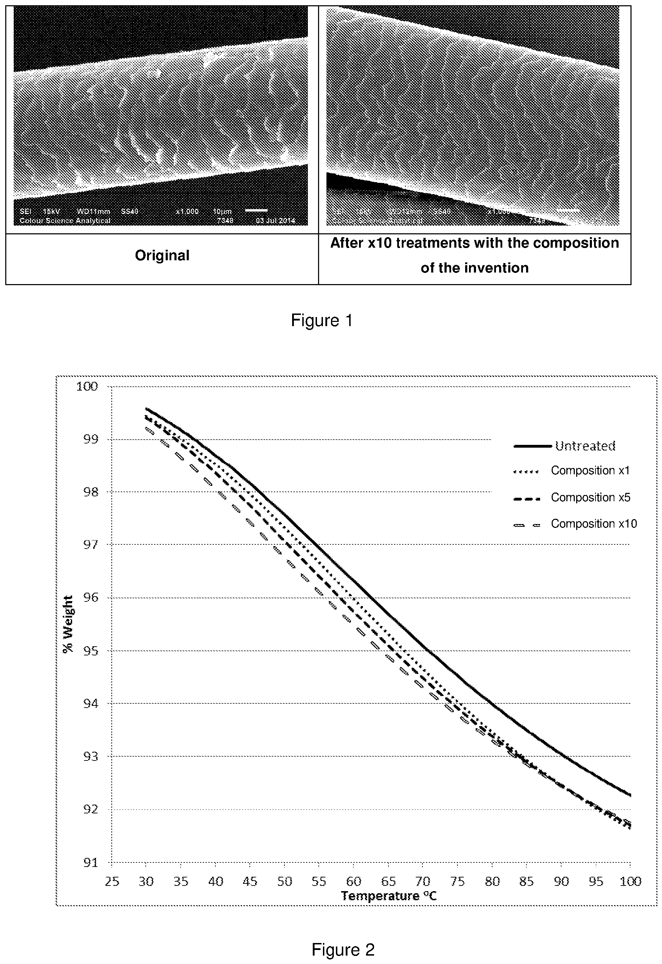

Several tresses of level 6 light brown hair were treated with the composition of Example 1 using the procedure of Example 1. The application procedure was repeated a number of times to observe the effects of multiple applications. These tresses were then analysed by Scanning Electron Microscopy (SEM) Energy Dispersive X-ray spectroscopy (EDX) and ThermoGravimetric Analysis (TGA). The results are shown in Figures 1 and 2.

The SEM results shown in Figure 1 demonstrate there is no visible physical difference between the two fibres. The fibre treated with the composition is in very good condition. The EDX data indicates that there is very little difference between the two fibres in terms of elemental composition on the surface of the fibre. Therefore it can be concluded that there is no residue left on the surface of the fibre after treatment with the conditioning composition of the invention.

The TGA results shown in Figure 2 demonstrate that there is a considerable difference in % weight loss in the fibres which have been treated with the composition of the invention compared to the untreated fibre as the temperature increases to 100°C. This %weight loss is greater on fibres which have had multiple applications of the composition of the invention.

This means that fibres treated with the composition are able to retain a larger amount of water than untreated fibres. It is known to those skilled in the art that retention of water provides reduced static and improved softness, shine, manageability and overall condition of the hair. Therefore, this data confirms that repeated applications of the composition of the present invention has led to reduced static and improved softness, shine, manageability and overall condition of the hair.

## Claims

1. The use of a composition comprising from 20 to 45 wt% of a water soluble ester of formula RCOXR' as a hair conditioning agent, wherein the hair conditioning agent is an ester of ethoxylated sorbitan and a fatty acid having from 10 to 30 carbon atoms which includes from 10 to 50 ethoxy groups.

2. The use according to claim 1 wherein the hair conditioning agent is an ester of alkoxylated sorbitan and an acid selected from stearic acid, lauric acid and oleic acid.

3. The use according to claim 1 or claim 2 wherein the hair conditioning agent is the compound of formula (I):

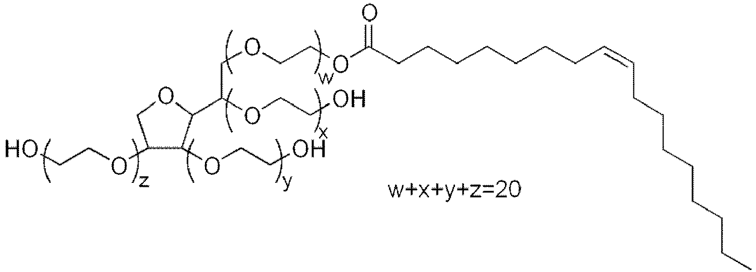

4. The use according to any preceding claim wherein the ester of formula RCOXR' and water together comprise at least 90 wt% of the composition.

5. The use according to any preceding claim which provides an improvement in condition of hair selected from one or more of an increase in softness, ease of handling, a reduction in static, a reduction in tangling, an increase in the ease of styling and "cuttability", increased shine, increased volume and increased strength.

## Patentansprüche

1. Verwendung einer Zusammensetzung, die 20 bis 45 Gew.-% eines wasserlöslichen Esters der Formel RCOXR' umfasst, als Haarkonditionierungsmittel, wobei es sich bei dem Haarkonditionierungsmittel um einen Ester von ethoxyliertem Sorbitan und einer Fettsäure mit 10 bis 30 Kohlenstoffatomen mit 10 bis 50 Ethoxygruppen handelt.

2. Verwendung nach Anspruch 1, wobei es sich bei dem Haarkonditionierungsmittel um einen Ester von alkoxyliertem Sorbitan und einer aus Stearinsäure, Laurinsäure und Ölsäure ausgewählten Säure handelt.

3. Verwendung nach Anspruch 1 oder Anspruch 2, wobei es sich bei dem Haarkonditionierungsmittel um die Verbindung der Formel (I) handelt:

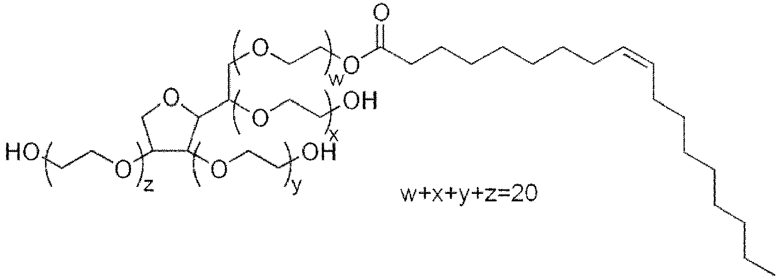

4. Verwendung nach einem der vorhergehenden Ansprüche, wobei der Ester der Formel RCOXR' und Wasser zusammen mindestens 90 Gew.-% der Zusammensetzung ausmachen.

5. Verwendung nach einem der vorhergehenden Ansprüche, die eine Verbesserung des Zustands von Haar bereitstellt, die aus einem oder mehreren von einer Erhöhung der Weichheit, der Handhabbarkeit, einer Verringerung der Statik, einer Verringerung der Verknotung, einer Erhöhung der Frisierbarkeit und Schneidbarkeit, erhöhtem Glanz, erhöhtem Volumen und erhöhter Festigkeit ausgewählt ist.

## Revendications

1. Utilisation d'une composition comprenant de 20 à 45% en poids d'un ester hydrosoluble de formule RCOXR' comme agent de conditionnement des cheveux, dans laquelle l'agent de conditionnement des cheveux est un ester d'un sorbitane éthoxylé et d'un acide gras ayant de 10 à 30 atomes de carbone, qui comporte de 10 à 50 groupements éthoxy.

2. Utilisation selon la revendication 1, dans laquelle l'agent de conditionnement des cheveux est un ester d'un sorbitane alcoxylé et d'un acide choisi parmi l'acide stéarique, l'acide laurique et l'acide oléique.

3. Utilisation selon la revendication 1 ou la revendication 2, dans laquelle l'agent de conditionnement des cheveux est le composé de formule (I) :

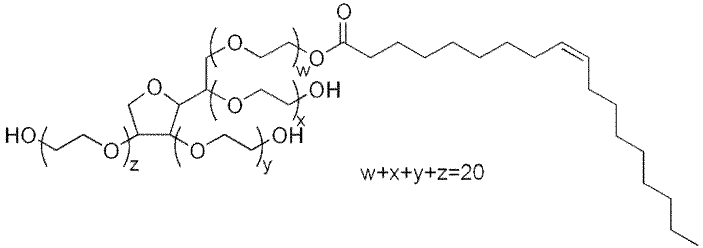

4. Utilisation selon l'une quelconque des revendications précédentes, dans laquelle l'ester de formule RCOXR' et l'eau constituent ensemble au moins 90% en poids de la composition.

5. Utilisation selon l'une quelconque des revendications précédentes, qui apporte une amélioration de l'état des cheveux choisie parmi une ou plusieurs parmi une augmentation de la douceur, une facilité de manipulation, une réduction du caractère électrostatique, une réduction de l'emmêlement, une augmentation de la facilité de coiffage et de « l'aptitude à être coupés », une augmentation du brillant, une augmentation du volume et une augmentation de la force.
